Europäisches Patentamt

European Patent Office

Office européen des brevets

(11) Publication number: **0 468 441 A1**

# EUROPEAN PATENT APPLICATION

(21) Application number: **91112320.6**

(22) Date of filing: **23.07.91**

(51) Int. Cl.⁵: **A61K  39/00**, A61K 39/12, A61K 39/21, A61K 37/02, A61K 39/395

(30) Priority: **23.07.90 IE 1427/90**

(43) Date of publication of application:
**29.01.92 Bulletin  92/05**

(84) Designated Contracting States:
**AT BE CH DE DK ES FR GB GR IT LI LU NL SE**

(71) Applicant: **Prendergast, Patrick Thomas Baybush**
**Straffan Kildare(IE)**

(72) Inventor: **Prendergast, Patrick Thomas Baybush**
**Straffan Kildare(IE)**

(74) Representative: **Gallo, Wolfgang, Dipl.-Ing. (FH) et al**
**Patentanwälte Dipl.-Ing. L. Fleuchaus, Dipl.-Phys. H. Schroeter, Dipl.-Ing K. Lehmann, Dipl.-Ing.W. Wehser, Dipl.-Ing.(FH) W. Gallo, Ludwigstrasse 26 W-8900 Augsburg(DE)**

(54) **Use of particular compounds for manufacture of pharmaceutical formulations for metabolic treatments.**

(57) There is provided the use of: 1. Group (A) Compounds - Arachidonic Acid binding peptides for the manufacture of pharmaceutical formulations for use as antigen to stimulate a therapeutic antibody response which can inactivate disease causing agents in the form of viruses, bacteria or mycoplasma; 2. Group (B) Compounds - Antibodies, either mono or polyclonal, produced to compounds of Group (A) for the manufacture of pharmaceutical formulations for administration to a patient (animal or human), particularly where the immune response would not be considered capable of producing enough antibodies to antigens of Group (A); 3. Group (C) Compounds - Unsaturated Fatty Acids, of carbon chain length greater than 18, which have been shown to have a greater affinity for Arachidonic Acid binding peptides than Arachidonic Acid itself, for example, docosahexaenoic acid and eicosapentaenoic acid, for the manufacture of pharmaceutical formulations. These fatty acids can be used as therapeutic agents in place of or in combination with compounds of Group (A) and/or Group (B).

1. Technical Field

This invention relates to use of: 1. Group (A) Compounds - Arachidonic Acid binding peptides for the manufacture of pharmaceutical formulations containing antigen to stimulate a therapeutic antibody response which can inactivate disease causing agents in the form of viruses, bacteria or mycoplasma; 2. Group (B) Compounds - Antibodies, either mono or polyclonal, produced to compounds of Group (A) for the manufacture of pharmaceutical formulations for administration to a patient (animal or human), particularly where the immune response would not be considered capable of producing enough antibodies to antigens of Group (A); 3. Group (C) Compounds - Unsaturated Fatty Acids, of carbon chain length greater than 18, which have been shown to have a greater affinity for Arachidonic Acid binding peptides than Arachidonic Acid itself, for example, docosahexaenoic acid and eicosapentaenoic acid for the manufacture of pharmaceutical formulations for metabolic treatment. These fatty acids can be used as therapeutic agents in place of or in combination with compounds of Group (A) and/or Group (B).

2. Background Art

In the past, vaccines or diseases caused by viruses have relied either on inactivated virus or on live attenuated virus. This can be illustrated by reference to the history of vaccines against poliomyelitis which began in the 1950's with the Salk vaccine. The Salk vaccine was prepared by growing poliovirus in tissue culture and then inactivating the virus with formaldehyde. The resulting inactivated or dead virus is administered by injection and appears to stimulate circulating antibodies which can neutralise the virus. In the case of the polio virus where there are three types, a trivalent vaccine is generally injected in order to immunise against all three types. Apart from the expense of preparing this kind of vaccine, it also has the disadvantage that it is made using a potentially virulent virus and there is a delicate margin between rendering the virus non-infectious while at the same time retaining its immunogenicity. In rare cases, the polio vaccine has actually caused polio. Also, in the case of the AIDS virus where many strains exist, the production of a multivalent vaccine would be very expensive.

An alternative vaccine type was subsequently developed, where the virus was cultured in cell lines until it lost its ability to cause disease - it became attenuated. This live attenuated virus is administered orally and induces a protective antibody response. This type of vaccine also has the disadvantage of being expensive since each batch of vaccine has to be extensively tested in animals. In addition, (a) the vaccine virus can, very occasionally, revert to virulence, causing disease, and (b) the attenuated virus can often die, thus failing to produce the desired immune response.

There is, therefore, a need for a vaccine which

(a) is simple and inexpensive to produce,
(b) does not employ any virus(es) or part thereof,
(c) induces an appropriate immune response,
(d) does not risk producing the corresponding disease. Herein is described an invention by which these requirements are met.

In accordance with convention, the following abbreviations are used for the amino acids:

| | |
|---|---|
| Alanine | Ala |
| Arginine | Arg |
| Asparagine | Asn |
| Aspartic Acid | Asp |
| Cysteine | Cys |
| Glutamine | Gln |
| Glutamic Acid | Glu |
| Glycine | Gly |
| Histidine | His |
| Isoleucine | Ile |
| Leucine | Leu |
| Lysine | Lys |
| Methionine | Met |
| Phenylalanine | Phe |
| Proline | Pro |
| Serine | Ser |
| Threonine | Thr |
| Tryptophan | Trp |

| Tyrosine | Tyr |
| Valine | Val |

## SUMMARY OF THE INVENTION

The present invention stems from the novel discovery that certain peptide/glycopeptide sequences exhibited as components of viral, bacterial and/or mycoplasma coat proteins or glycoproteins act in a deleterious manner to human and animal biological functions by binding specifically to Arachidonic Acid (AA) molecules. Binding also takes place with associated polyunsaturated fatty acids such as Docosahexaenoic Acid (DHA) or Eicosapentaenoic Acid (EPA) (Omega-3 fatty acids). The presence of these peptides, polypeptides/glycopeptide sequences which facilitate Arachidonic Acid binding is the essential factor which allows these classes of viruses, bacteria and mycoplasma to attach and/or infect host cells. This discovery opens new avenues to manufacture of pharmaceutical formulations for treatment and/or prevention by inoculation or therapy of immunosuppressive disorders and/or viral, certain bacterial and/or mycoplasma infections.

This invention provides both natural and synthetic or recombinant peptides, polypeptides/glycopeptides suitable for manufacture of pharmaceutical formulations for therapy and/or vaccination against indications/disease caused by foreign bodies.

This invention relates to manufacture of pharmaceutical formulations for treatment of persons and animals with indications of immuno-deficiency, wherein the said indication is resultant from viral and/or retroviral infection and/or infectious protein units. The invention further relates to manufacture of pharmaceutical formulations for treatments for establishing improved immuno response for persons and prophylactic treatment for persons where immuno-malfunction due to infection is considered a future risk. The invention also relates to manufacture of pharmaceutical formulations for treatments of indications resultant from phospholipase and/or lipoxygenase and/or cyclooxygenase pathway interference within the body causing alteration in normal levels of leukotrienes and/or prostaglandins. This interference (phospholipase/lipoxygenase/cyclooxygenase) resulting from the binding of viral/bacterial/mycoplasma protein/glycoprotein (data generated showing such interference is presented herewith) with Arachidonic Acid or one of its metabolites resulting in the alteration of leukotrienes and/or prostaglandins secretion by macrophages and other Lymphocytes.

The invention further relates to manufacture of pharmaceutical formulations for administration of one or more mono/poly-clonal antibodies specific to the viral protein sequence or sequences which bind Arachidonic Acid or other polyunsaturated fatty acids (e.g., Docosahexaenoic Acid (DHA) or Eicosapentaenoic Acid (EPA) (Omega-3 fatty acids). The invention also relates to manufacture of pharmaceutical formulations for the administration of peptides, polypeptides/glycopeptides and/or mono/multi-clonal antibodies to the specific peptide sequences that bind Arachidonic Acid or Docosahexaenoic Acid (DHA) or Eicosapentaenoic Acid (EPA) (Omega-3 fatty acids). In one embodiment, the invention relates to use of monoclonal and/or poly-clonal antibodies to one or more of the sequences of alpha feto protein (AFP/human, animal or synthetic or recombinant) which are specific in causing the binding of alpha feto protein (AFP) to Arachidonic Acid for the manufacture of pharmaceutical formulations. In one embodiment of the present invention, the host organism (i.e., man or animal) is administered with either antibodies to alpha feto protein (AFP/human, animal or synthetic or recombinant) or antibodies to specific amino acid peptide sequences, in particular the sequences which attach alpha feto protein (AFP/human, animal or synthetic or recombinant) and/or the HIV viral coat protein p24 (and in particular, but not limited to, the p24 amino acid sequence Ala-Asn-Pro-Asp-Cys-Lys-Thr-Ile-Leu-Lys-Ala-Leu-Gly-Pro-Ala-Ala-Thr-) and/or HIV viral coat protein gp120 (and in particular, but not limited to, the gp120 amino acid sequence Cys-Thr-His-Gly-Ile-Arg-Pro-Val-Val-Ser-Thr-Gln-Leu-Leu-Asn-Gly-Ser-Leu-Ala-Glu-) and/or HIV viral coat protein gp41(and in particular, but not limited to, the gp41 amino acid sequences Leu-Gly-Leu-Trp-Gly-Cys-Ser-Gly-Lys-Ile-Cys, Leu-Gly-Ile-Trp-Gly-Cys-Ser-Gly-Lys-Leu-Ile-Cys, Leu-Gly-Ile-Trp-Gly-Cys-Ser-Gly-Lys-His-Ile-Cys, Leu-Gly-Met-Trp-Gly-Cys-Ser-Gly-Lys-His-Ile-Cys, or Leu-Asn-Ser-Trp-Gly-Cys-Ala-Phe-Arg-Gln-Val-Cys) and/or HIV viral coat protein gp160 to Arachidonic Acid.

In another embodiment, the host organism (man or animal) is immunised with alpha feto protein of human, animal or synthetic or recombinant origin, which may be in combination with a suitable adjuvant, to develop the said antibodies so that if any specific cell type or a combination of cell types of the said host organism is challenged by a foreign body (i.e., viral/bacterial/mycoplasma infection), using the said sequence or a similar peptide sequence to attach to Arachidonic Acid in whole or in part (as a mechanism by which the said disease causing organism can attach to the host cell and cause infection or alter the host body's immune system), the foreign body will be restricted, either partially or totally, from attaching to the host cells. This will result in preventing the infecting organism from attaching or interacting with the host tissue. Such immunity will be gained by vaccinating with the peptide of alpha feto protein (human, animal or synthetic or recombinant) or parts thereof - such parts being the binding sequences of AFP with Arachidonic Acid.

Compounds used for the manufacture of pharmaceutical formulations according to this invention include those of GROUPS A, B an C (defined below), and the pharmaceutical formulations are administered as treatments against viral, bacterial, and mycoplasma infections by any suitable route including enteric, parenteral, topical, oral, rectal, nasal or vaginal routes. Parenteral routes include subcutaneous, intramuscular, intravenous and sublingual administration. The preferred route of administration would be an intravenous one but this may not be feasible with a large patient base and oral administration of compounds of GROUP A, B or C may be the most preferred route.

The present invention further provides use of any compound of GROUP A, B or C in the manufacture or preparation of formulations, and especially pharmaceutical formulations, for use in treatments against HIV/HTLV-I,II,III and other viral diseases and diseases caused by mycoplasma or bacteria. The invention also provides the pharmaceutical formulations themselves and processes for preparing the pharmaceutical formulations.

The present invention also relates to a process of using the novel phenomenon associated with p24 binding of Arachidonic Acid/Glass complex to remove HIV p24 antigens from a patient's plasma or as a method to establish a quantitative assay for HIV p24 antigen levels in a plasma.

The present invention also relates to a method comprising administering as a treatment to blood plasma or transplant organs or stored semen, a formulation containing in whole or in part a peptide sequence or Omega-3 fatty acid which binds the attachment site on Alpha feto protein or HIV gp120/gp41/gp160/p24 for Arachidonic Acid for the removal of infectious organisms (viruses/bacterial/mycoplasma) either in the free state or as infected host cells.

The present invention further relates to a method for treating virus, mycoplasma or bacterial organisms, comprising genetically manipulating DNA or RNA sequences of the virus, mycoplasma or bacteria, which genetic manipulation will alter the glycopeptide sequences present in the coat or membrane of the virus, mycoplasma or bacterial organism, so as to inhibit the ability of the virus, mycoplasma or bacterial organism from binding Arachidonic Acid free or exposed on the host cell membrane, such alterations rendering virions and mycoplasma utilizing such host cell binding incapable of active attachment or infection.

The present invention also relates to manufacture of pharmaceutical formulations for inoculating into a patient a human, animal or synthetic or recombinant amino acid sequence with or without adjuvant, to produce an antibody response, the antibodies being mono or polyclonal, and causing blocking and shielding by binding to binding sites exposed by infectious organisms or parts thereof for the fatty acid, Arachidonic Acid, or associated Omega-3 fatty acids, and rendering further transmission and or infection impossible.

The present invention also relates to administering compounds from Group A, Group B or Group C prior to, while or after administering anti-viral, antimycoplasmic or antibacterial agents to prevent immunosuppression which is caused when Arachidonic Acid binding sites present on these viruses, mycoplasma or bacteria are released by the use of anti-viral, antimycoplasma or antibacterial agents.

The present invention further relates to the use of Arachidonic Acid or its analogues or other compounds, or combinations thereof, as decoy molecules attached to the surface membranes of liposomes, red blood cells or other transport vehicles for the manufacture of pharmaceutical formulations for use as therapeutic agents for the removal, by adherence, of active free virus, mycoplasma or bacteria, or combinations thereof, or infected host cells expressing viral coat proteins or glycopeptides.

The present invention also relates to the use of compounds of Group A, Group B or Group C for the treatment outside the body of blood products, semen or organs.

The present invention further relates to use of compounds which are inhibitors of phospholipase A2, cyclooxygenase and lipoxygenase in combination with compounds of Group A, Group B or Group C for manufacture of pharmaceutical formulations for use in improving the immune status of a patient suffering immuno-malfunction as a result of viral and/or mycoplasma interference with the ratio and/or proportion of Arachidonic Acid metabolites produced.

Pharmaceutical formulations prepared according to the invention include the compound of GROUP A, B or C contained in a gelatine capsule, in tablet form, dragee, syrup, suspension, topical cream, suppository, injectable solution, or kits for the preparation of syrups, suspension, topical cream, suppository or injectable solution just prior to use. Also, compounds of GROUP A, B or C may be included in composites which facilitate their slow release into the blood stream, e.g., silicon discs, polymer beads, or implants.

Pharmaceutical formulations prepared according to the invention include any of the compounds of GROUPS A, B or C contained in liposomes to enhance the potency and/or safety of the formulation.

Compounds of GROUP A, B or C, particularly antibodies to alpha feto protein (AFP/human, animal or synthetic or recombinant) or portions thereof which bind to Arachidonic Acid (AA), may be used to target drugs carried in liposomes with the mono/polyclonal antibodies to AFP used to bind the drug laden liposomes specifically with virus infected cells.

Pharmaceutical formulations prepared according to the invention include the employment of compounds of GROUP A, B or C in admixture with conventional excipients, that is, pharmaceutically acceptable organic or inorganic carrier substances which do not deleteriously react with the active compounds. Suitable pharmaceutically acceptable carriers include, but are not limited to, water, salt solutions, alcohols, gum arabic, vegetable oils, gelatine, carbohydrates, magnesium stearate, talc, silicic acid, viscous paraffin, fatty acid mono- and diglycerides, etc. The preparative procedure may include the sterilisation of the pharmaceutical preparations. The compounds may be mixed with auxiliary agents such as adjuvants (Freund's Complete adjuvant/Alum etc.), lubricants, preservatives, stabilisers, salts for influencing osmotic pressure, etc., which do not react deleteriously with the compounds.

The dosage of any one or more of compounds from GROUP A, B or C which is effective in treatment against infectious agents will depend on many factors, including the specific compound or combination of compounds being utilised, the mode of administration, and the organism being treated. Dosages of a particular compound or combinations of compounds, each belonging to that class defined by GROUP A, B or C, for a given host can be determined using conventional considerations; for example, by customary comparison of the differential activities of the subject compounds and of a known agent, by means of an appropriate pharmacological protocol. Further, the effectiveness of a particular regimen can be monitored by following over time the presence of viral/bacterial/mycoplasma particles in blood samples of the host being treated or by monitoring the plasma levels of free Arachidonic Acid, which are direct indications of Phospholipase A2 activation, and which levels have been shown to increase following viral infection. Furthermore, after inoculation, levels of antibodies to alpha feto protein or other similar acting peptides may be used to determine the effectiveness of the vaccination.

In one aspect of the invention, a unit dose comprises 0.01 to 1000 mg of a formulation comprised of a compound selected from GROUP A, B or C.

In one embodiment of the invention, the pharmaceutical formulation is administered orally in unit doses once per day when the compound is in a slow release form or up to eight unit doses per day when the compound is in its native form. Alternatively or additionally, the pharmaceutical formulation is administered intravenously in unit doses comprising a compound from GROUP A, B or C in the range of 0.001 mg to 450 mg per Kg of body weight.

In another embodiment of the invention, the method includes the step of treating a patient with an adjuvant either prior to and/or simultaneously with and/or after, administering a pharmaceutical formulation comprising a compound from GROUP A or B.

## GROUP A, B OR C COMPOUNDS

Amino Acid sequence, in whole or in part, of Alpha Feto Protein (Human, animal or synthetic or recombinant). Peptide sequence(s) which are similar to the peptide sequence(s) which are active in the binding of alpha feto protein (AFP/human, animal or synthetic or recombinant) with Arachidonic Acid.

Amino acid sequence of any virus coat protein or glycoprotein which utilizes the binding of Arachidonic Acid as a means of attachment and infection and/or initially the process that creates host immune malfunction by distorting the metabolic fate of Arachidonic Acid regarding prostaglandins and leukotriene imbalance.

Peptide sequence(s) which are similar to the peptide sequence(s) which are active in the binding of the membrane of Mycoplasma with Arachidonic Acid.

Peptide sequence(s) which are similar to the peptide sequence(s) which are active in the binding of alpha feto protein (AFP/human, animal or synthetic or recombinant) with Omega-3 fatty acids. Peptide sequence(s) which are similar to the peptide sequence(s) which are active in the binding of the membrane of Mycoplasma with Omega-3 fatty acids.

Peptide sequence(s) which are similar to the peptide sequence(s) which are active in the binding of alpha feto protein (AFP/human, animal or synthetic or recombinant) with polyunsaturated fatty acids. These unsaturated fatty acids are classified as having a greater affinity for binding AFP/HIV gp120, gp41 and gp160 type molecules than free or membrane bound Arachidonic Acid (AA).

Peptide sequences(s) which are similar to the peptide sequence(s) which are active in the binding of the membrane of Mycoplasma with polyunsaturated fatty acids. These unsaturated fatty acids are classified as having a greater affinity for binding AFP/HIV gp120, gp41 and gp160 type molecules than free or membrane bound Arachidonic Acid (AA).

Peptide sequence(s) which are similar to the peptide sequence(s) which are active in the binding of alpha feto protein (AFP/human, animal or synthetic or recombinant) with Eicosapentaenoic Acid (EPA).

Peptide sequence(s) which are similar to the peptide sequence(s) which are active in the binding of the membrane of Mycoplasma with Eicosapentaenoic Acid (EPA).

Peptide sequence(s) which are similar to the peptide sequence(s) which are active in the binding of alpha feto protein (AFP/human, animal or synthetic or recombinant) with Docosahexaenoic Acid (DHA).

Peptide sequence(s) which are similar to the peptide sequence(s) which are active in the binding of the membrane of Mycoplasma with Docosahexaenoic Acid (DHA).

Peptide sequence(s) which are similar to the peptide sequence(s) which are active in the binding of HIV viral coat protein gp120 (and in particular, but not limited to, the gp120 amino acid sequence Cys-Thr-His-Gly-Ile-Arg-Pro-Val-Val-Ser-Thr-Gln-Leu-Leu-Leu-Asn-Gly-Ser-Leu-Ala-Glu-) with Arachidonic Acid.

Peptide sequence(s) which are similar to the peptide sequence(s) which are active in the binding of HIV viral coat protein gp120 (and in particular, but not limited to, the gp120 amino acid sequence Cys-Thr-His-Gly-Ile-Arg-Pro-Val-Val-Ser-Thr-Gln-Leu-Leu-Leu-Asn-Gly-Ser-Leu-Ala-Glu-) with Omega-3 fatty acids.

Peptide sequence(s) which are similar to the peptide sequence(s) which are active in the binding of HIV viral coat protein gp120 (and in particular, but not limited to, the gp120 amino acid sequence Cys-Thr-His-Gly-Ile-Arg-Pro-Val-Val-Ser-Thr-Gln-Leu-Leu-Leu-Asn-Gly-Ser-Leu-Ala-Glu-) with polyunsaturated fatty acids. These unsaturated fatty acids are classified as having a greater affinity for binding AFP/HIV gp120, gp41 and gp160 type molecules than free or membrane bound Arachidonic Acid (AA).

Peptide sequence(s) which are similar to the peptide sequence(s) which are active in the binding of HIV viral coat protein gp120 (and in particular, but not limited to, the gp120 amino acid sequence Cys-Thr-His-Gly-Ile-Arg-Pro-Val-Val-Ser-Thr-Gln-Leu-Leu-Leu-Asn-Gly-Ser-Leu-Ala-Glu-) with Eicosapentaenoic Acid (EPA).

Peptide sequence(s) which are similar to the peptide sequence(s) which are active in the binding of HIV viral coat protein gp120 (and in particular, but not limited to, the gp120 amino acid sequence Cys-Thr-His-Gly-Ile-Arg-Pro-Val-Val-Ser-Thr-Gln-Leu-Leu-Leu-Asn-Gly-Ser-Leu-Ala-Glu-) with Docosahexaenoic Acid (DHA).

Peptide sequence(s) which are similar to the peptide sequence(s) which are active in the binding of HIV viral coat protein p24 (and in particular, but not limited to, the p24 amino acid sequence Ala-Asn-Pro-Asp-Cys-Lys-Thr-Ile-Leu-Lys-Ala-Leu-Gly-Pro-Ala-Ala-Thr-) with Arachidonic Acid.

Peptide sequence(s) which are similar to the peptide sequence(s) which are active in the binding of HIV viral coat protein p24 (and in particular, but not limited to, the p24 amino acid sequence Ala-Asn-Pro-Asp-Cys-Lys-Thr-Ile-Leu-Lys-Ala-Leu-Gly-Pro-Ala-Ala-Th-r) with Omega-3 fatty acids.

Peptide sequence(s) which are similar to the peptide sequence(s) which are active in the binding of HIV viral coat protein p24 (and in particular, but not limited to, the p24 amino acid sequence Ala-Asn-Pro-Asp-Cys-LyS-Thr-Ile-Leu-Lys-Ala-Leu-Gly-Pro-Ala-Ala-Thr-) with polyunsaturated fatty acids. These unsaturated fatty acids are classified as having a greater affinity for binding AFP/HIV gp120, gp41 and gp160 type molecules than free or membrane bound Arachidonic Acid (AA).

Peptide sequence(s) which are similar to the peptide sequence(s) which are active in the binding of HIV viral coat protein p24 (and in particular, but not limited to, the p24 amino acid sequence Ala-Asn-Pro-Asp-Cys-Lys-Thr-Ile-Leu-Lys-Ala-Leu-Gly-Pro-Ala-Ala-Thr-) with Eicosapentaenoic Acid (EPA).

Peptide sequence(s) which are similar to the peptide sequence(s) which are active in the binding of HIV viral coat protein p24 (and in particular, but not limited to, the p24 amino acid sequence Ala-Asn-Pro-Asp-Cys-Lys-Thr-Ile-Leu-Lys-Ala-Leu-Gly-Pro-Ala-Ala-Thr-) with Docosahexaenoic Acid (DHA).

Amino Acid sequence, in whole or in part, of an Anti Serum to Alpha Feto Protein (Human, animal or synthetic or recombinant).

Anti Serum to the peptide sequence(s) which are active in the binding of alpha feto protein (AFP/human, animal or synthetic or recombinant) with Arachidonic Acid.

Anti Serum to the peptide sequence(s) which are active in the binding of alpha feto protein (AFP/human, animal or synthetic or recombinant) with Omega-3 fatty acids.

Anti Serum to the peptide sequence(s) which are active in the binding of alpha feto protein (AFP/human, animal or synthetic or recombinant) with polyunsaturated fatty acids. These unsaturated fatty acids are classified as having a greater affinity for binding AFP/HIV gp120/gp41/gp160 type molecules than free or membrane bound Arachidonic Acid (AA).

Anti Serum to the peptide sequence(s) which are active in the binding of alpha feto protein (AFP/human, animal or synthetic or recombinant) with Eicosapentaenoic Acid (EPA).

6

Anti Serum to the peptide sequence(s) which are active in the binding of alpha feto protein (AFP/human, animal or synthetic or recombinant) with Docosahexaenoic Acid (DHA).

Anti Serum to the peptide sequence(s) which are active in the binding of HIV viral coat protein gp120 (and in particular, but not limited to, the gp120 amino acid sequence Cys-Thr-His-Gly-Ile-Arg-Pro-Val-Val-Ser-Thr-Gln-Leu-Leu-Leu-Asn-Gly-Ser-Leu-Ala-Glu-) with Arachidonic Acid.

Anti Serum to the peptide sequence(s) which are active in the binding of HIV viral coat protein gp120 (and in particular, but not limited to, the gp120 amino acid sequence Cys-Thr-His-Gly-Ile-Arg-Pro-Val-Val-Ser-Thr-Gln-Leu-Leu-Leu-Asn-Gly-Ser-Leu-Ala-Glu-) with Omega-3 fatty acids.

Anti Serum to the peptide sequence(s) which are are active in the binding of HIV viral coat protein gp120 (and in particular, but not limited to, the gp120 amino acid sequence Cys-Thr-His-Gly-Ile-Arg-Pro-Val-Val-Ser-Thr-Gln-Leu-Leu-Leu-Asn-Gly-Ser-Leu-Ala-Glu-) with polyunsaturated fatty acids. These unsaturated fatty acids are classified as having a greater affinity for binding AFP/HIV gp120, gp41 and gp160 type molecules than free or membrane bound Arachidonic Acid (AA).

Anti Serum to the peptide sequence(s) which are are active in the binding of HIV viral coat protein gp120 (and in particular, but not limited to, the HIV gp120 amino acid sequence Cys-Thr-His-Gly-Ile-Arg-Pro-Val-Val-Ser-Thr-Gln-Leu-Leu-Leu-Asn-Gly-Ser-Leu-Ala-Glu) with Eicosapentaenoic Acid (EPA).

Anti Serum to the peptide sequence(s) which are active in the binding of HIV viral coat protein gp120 (and in particular, but not limited to, the gp120 amino acid sequence Cys-Thr-His-Gly-Ile-Arg-Pro-Val-Val-Ser-Thr-Gln-Leu-Leu-Leu-Asn-Gly-Ser-Leu-Ala-Glu-) with Docosahexaenoic Acid (DHA).

Anti Serum to the peptide sequence(s) which are active in the binding of HIV viral coat protein p24 (and in particular, but not limited to, the p24 amino acid sequence Ala-Asn-Pro-Asp-Cys-Lys-Thr-Ile-Leu-Lys-Ala-Leu-Gly-Pro-Ala-Ala-Thr-) with Arachidonic Acid.

Anti Serum to the peptide sequence(s) which are active in the binding of HIV viral coat protein p24 (and in particular, but not limited to, the p24 amino acid sequence Ala-Asn-Pro-Asp-Cys-Lys-Thr-Ile-Leu-Lys-Ala-Leu-Gly-Pro-Ala-Ala-Thr-) with Omega-3 fatty acids.

Anti Serum to the peptide sequence(s) which are active in the binding of HIV viral coat protein p24 (and in particular, but not limited to, the p24 amino acid sequence Ala-Asn-Pro-Asp-Cys-Lys-Thr-Ile-Leu-Lys-Ala-Leu-Gly-Pro-Ala-Ala-Thr-) with polyunsaturated fatty acids. These unsaturated fatty acids are classified as having a greater affinity for binding AFP/HIV gp120/gp41/gp160 type molecules than free or membrane bound Arachidonic Acid (AA).

Anti Serum to the peptide sequence(s) which are active in the binding of HIV viral coat protein p24 (and in particular, but not limited to, the p24 amino acid sequence Ala-Asn-Pro-Asp-Cys-Lys-Thr-Ile-Leu-Lys-Ala-Leu-Gly-Pro-Ala-Ala-Thr-) with Eicosapentaenoic Acid (EPA).

Anti Serum to the peptide sequence(s) which are active in the binding of HIV viral coat protein p24 (and in particular, but not limited to, the p24 amino acid sequence Ala-Asn-Pro-Asp-Cys-Lys-Thr-Ile-Leu-Lys-Ala-Leu-Gly-Pro-Ala-Ala-Thr-) with Docosahexaenoic Acid (DHA).

Docosahexaenoic Acid (DHA)

Eicosapentaenoic Acid (EPA)

Polyunsaturated fatty acids which have a greater affinity for binding AFP/HIV gp120, gp41 and gp160 type molecules than free or membrane bound Arachidonic Acid (AA).

Omega-3 fatty Acids.

## DETAILED DESCRIPTION OF THE INVENTION

Alfa feto protein (AFP) is a major serum protein (Mol. wt. = 70,000) synthesised during fetal life. Reappearance of AFP in adult serum often signals pathological conditions, particularly hepatocarcinomas and teratocarcinomas. In contrast, albumin increases steadily during fetal and neonatal growth and shows no apparent changes in concentration associated with development of liver and germ cell tumors.

Protein Structure of Human AFP: The molecular weight of mature human AFP is calculated to be 66,300 without carbohydrate and 69,063 with 4% carbohydrate. These figures are in agreement with those obtained by analysis of AFP polypeptides by conventional means.

A comparison of the amino acid sequences of human AFP and mouse AFP: The overall identity is 66% with the highest identity found in domain 3 (72%), followed by domain 2(67%) and domain 1(59%) (see Table 1 below). Similar identities are found between human AFP and rat AFP (Table 1). All three AFPs share a long stretch of sequence in domain 3 (residues 510-564) with an 87% identity between human AFP and mouse or rat AFP and 95% between mouse and rat AFPs.

Comparisons of amino acid sequences between human AFP and human albumin: The overall identity was 39% (Table 1) with, as in the case of AFP, the highest identify found in domain 3 (48%), followed by domain 2 (40%) and domain 1 (30%) (Table 1). The similarity at the $NH_2$-terminal region is particularly low, showing only a 16% identity at residues 1086.

The secondary structure of human AFP, as predicted from the amino acid sequence according to the procedure of Chou and Fasman is very similar to that of mouse AFP but significantly different from that of human albumin.

Human AFP is the second mammalian AFP whose primary structure has been elucidated in its entirety from DNA clones. Mature human AFP contains 590 amino acids; mouse AFP contains 586. The four extra amino acid residues are found in domain 1. This domain has the least amino acid sequence homology and it may be speculated that species differences in the AFP function may be attributed to this domain. For instance, the binding of estrogen may occur at domain 1, because this activity is reported to be specific to mouse and rat AFPs.

Table 1. Nucleotide and amino acid sequence comparisons between human AFP and mouse AFP (MAFP), rat AFP (RAFP), and human albumin (HSA)

| Region | Positions | Identical nucleotides, % | | | Positions | Identical amino acids, % | | |
|---|---|---|---|---|---|---|---|---|
| | | MAFP | RAFP | HSA | | MAFP | RAFP | HSA |
| 5' noncoding | 1-44 | 57 | — | 36 | | | | |
| Signal peptide | 45-101 | 63 | — | 60 | -19 to -1 | 47 | — | 53 |
| Domain 1 | 102-692 | 71 | (72) | 45 | 1 to 197 | 59 | (59) | 30 |
| Domain 2 | 693-1,268 | 78 | 74 | 54 | 198 to 389 | 67 | 65 | 40 |
| Domain 3 | 1,269-1,871 | 78 | 76 | 57 | 390 to 590 | 72 | 74 | 48 |
| 3' noncoding | 1,872-2,029 | 64 | 57 | 48 | | | | |
| Overall (mature protein) | | 75 | (74) | 52 | | 66 | (67) | 39 |

Figures in parentheses are calculated on the basis of the known sequence of rat AFP.

In contrast to domain 1, domain 3 of human AFP exhibits a high degree of amino acid sequence homology to mouse AFP. In particular, the sequence covering amino acid residues 510-564 shows an 87% identity. AFP is shown to have a high affinity to unsaturated fatty acids.

Human AFP shows a 39% overall amino acid sequence identity to human albumin. It is noteworthy that the majority of the homologous amino acid residues are the same as those conserved in mouse AFP. This suggests that these amino acid residues are important in preserving basic structural features common to AFP and albumin.

There is one potential site in human AFP at which N-glycosylation occurs, in agreement with chemical analysis. In mouse and rat AFPs, there are three potential N-glycosylation sites, one being at the same location as in human AFP. The second site is found in domain 2 in both species, and the third is located in domain 3 in mouse AFP but in domain 1 in rat AFP. Conservation of the carbohydrate attachment sites in human, mouse and rat AFPs suggests that this moiety may play an important role, possibly in catabolism of AFP molecules.

By screening the viral peptides and glycopeptides for their ability to bind Arachidonic Acid and for their ability to activate phospholipase enzymes in the membranes of target cells, we have been able to demonstrate that binding Arachidonic Acid followed by activation of phospholipase enzymes is one of the principle prerequisites to establishing infection, and after infection, the breakdown products from phospholipase A2 (PLA2) activity which is Arachidonic Acid is bound preferentially by certain viral peptides and brought down a metabolic route to HPETE and leukotrienes which increase T8 and decrease T4 cell numbers causing immunosuppression.

It will be obvious that this patent presents a revolutionary treatment which does not have several of the inherent risks of conventional vaccines. Because of the design of the patent's pharmaceutical treatments, the development of viral mutant strains does not present difficulties to the effectiveness of the treatment outlined. This patent's pharmaceutical treatments not only avoid the risk of using viruses but also eliminate the need to use specific viral antigens.

Regardless of the methods used to produce a vaccine, the sought-for properties are all the same. Obviously, the vaccine must be effective at stimulating the immune system, producing an immunity that is as long-lived as possible.

Virus infection may have profound effects on the immune system. The classical observation is the loss of tuberculin test positively during measles infections. The tuberculin test is a DTH reaction and in this instance is used as a matter of T cell reactivity.

Some of these effects may be due to infection of the lymphoid and phagocytic cells, while others may be secondary to the release of mediators with powerful non-specific activities.

Immunological triggers (e.g., antigen or anti-IgE) perturb the mast cell membrane, leading to calcium ion influx. Changes in the plasma membrane associated with activation allow phospholipase A to release Arachidonic Acid which can then be metabolised by lipoxygenase or cyclooxygenase enzymes, depending on the mast cell type. The newly synthesised products include prostaglandin A and tromboxane A (Cyclooxygenase pathway), and SRS (which is 15-HPETE leukotriene LTC + LTD) and chemotactic LTB (lipoxygenase pathway).

The calcium ion influx has two main results. Firstly, there is an exocytosis of granule contents with the release of preformed mediators, the major one being histamine. Secondly, there is the induction of synthesis of newly formed mediators from Arachidonic Acid leading to the production of prostaglandins and leukotrienes.

It is becoming clear that different newly-formed mediators arise from the different populations of mast cells. Thus, the different clinical effects in different organs may be related to the varying population of mast cells in each.

There are at least two sub-populations of mast cells, the MMC and CTMC. The differences in the morphology and pharmacology suggest different functional roles in-vivo. MMCs are associated with parasitic worm infections and possibly allergic reactions. In comparison to the CTMC, the MMC is smaller, shorter lived, T cell dependent, has more surface Fc receptors and contains intracytoplasmic IgE. Both cells contain histamine and serotonin in their granules; the higher histamine content of the CTMC may be accounted for by the greater number of granules. Major Arachidonic Acid metabolites, prostaglandins and leukotrienes, are produced by both mast cell types but in different amounts. For example, the ratios of production of the leukotriene LTC4 to the prostaglandin PGD is 25:1 in the MMC and 1:40 in the CTMC.

In addition to the above, the present inventor found that viral proteins, and in particular the HIV AIDS virus coat proteins gp120/gp41/gp160, cause binding to Arachidonic Acid and irregularities in the production of leukotrienes and prostaglandins. Resulting from this novel discovery, it was found that compounds which inhibit the phospholipase A2 enzyme, cyclooxygenase and lipoxygenase pathways of Arachidonic Acid can be administered as part of a treatment regime in humans or animals.

It is clear from the foregoing that the production of leukotrienes and prostaglandins play a vital role in the production of both T4 and T8 cells within the body. In any autoimmune response, these specific T cells will determine the overall ability of the body to defend and overcome infection. In previous treatments of viral infection, it has always been the case that the treatment solely consisted of anti-viral agents either used alone or in conjunction with immune modulators. This present patent presents pharmaceutical formulations for treatments, which either alone or in conjunction with anti-viral and/or immune modulators, in which the main mode of action is to regulate normal production of T4 and/or T8 cells. This mode of action of the present patent's drug treatment is to counteract the action of viral coat protein in causing the immune defence system to function in a manner deleterious to itself. In the case of AIDS patients, it has been shown that the patient's immune system does not function properly in defending itself against the virus mainly because the ratio of T4 to T8 cells is not correct. Douglas L. Myers et al (Progression of HIV-1 disease in a population of seropositive Active Duty Navy and Marine Corps Personnel IV International Conference on AIDS (4149) 1988) determined the rate of HIV-1 diseases progression in 490 HIV-1 seropositive personnel. Of these 490 patients initially on the trial, 100 were followed for over 12 months. The patients were classified using a modified Walter Reed classification. He concluded that at 12 to 24 months follow-up, 58% of patients remained at the same stage, 32% progressed to a higher stage and 10% showed regression (within the confines of the classification system used). During this interval, the patient's T4 cell count also declined an average of 10% in those patients with apparent regression. V. Carcaba et al (Epidemiological, Clinical and Immunological experience with HIV infection in Different Risk Populations in the North of Spain - IV International Conference on AIDS (4164) 1988) showed from a five year hospital experience that antibody prevalence and disease severity were inversely related to the number of T-helper cells and T4/T8 ratio.

D. Israel-Biet et al (The Laennec Working Staging System for Classification of HIV+ Patients - Laennec Study Group Paris - IV International Conference on AIDS patients based on a study of 361 HIV+ patients for a period of 30 months. This system is known as the Laennec Working Staging System (LWSS).

The three classifications are as follows:

| NO. OF T4 CELLS | NON AIDS | | | AIDS | | | |
|---|---|---|---|---|---|---|---|
| | A | B | C | I | K | E | L |
| I  T4>600 | 70 | 66 | 8 | 1 | | | |
| II  300<T4<600 | 30 | 56 | 14 | 1 | 1 | | |
| III T4<300 | 12 | 18 | 21 | 46 | 13 | 1 | 3 |

(A=Asymptomatic,  B=Lymphadenopathy,  C=Constitutional symptoms, I=Opportunistic infection, K=Kaposi's sarcoma, E=Encephalopathy, L=Lymphoma).

It can be observed from this data that as the T4 cells reduced, the range of indication increased, resulting in greater danger to the patients.

This present patent presents work which shows that HIV coat proteins are able to bind several molecules such as polyunsaturated nonesterified fatty acids. Among the latter, Arachidonic Acid (AA), the major precursor for prostaglandin and leukotriene synthesis, was found to bind to viral genome proteins and in particular to HIV gp120, gp41, p24, gp 160. In particular, the present patent presents work which shows that AFP also binds AA in a similar manner to the HIV viral coat proteins. Moreover, HIV viral coat proteins are able to inhibit AA metabolism in cell free systems. They were shown to increase the recruitment of AA from phospholipids and to decrease the synthesis of prostaglandins. Macrophages are the major source of Arachidonic Acid metabolites, including prostaglandins (PG) produced by the cyclooxygenase pathway and leukotrienes (LT) produced by the lipoxygenase pathway. There has been much interest in the role of PG as macrophage soluble suppressor factors. PG have been shown to inhibit interleukin 2 (IL-2 production by T cells, probably by inducing the activation of suppressor T cells which in turn inhibit IL-2-producing T cells. LT were also shown to regulate T-cell proliferation.

In order to clarify the relationships between the in-vitro immunosuppressive effects of viral coat proteins (VCP) and the different suppression mechanisms induced by PG and LT, the possible influence of viral coat protein (VCP) on Arachidonic Acid (AA) metabolism in the macrophage - cell line was investigated.

**METHOD**

Labeling and analysis of cellular lipids: Macrophages were incubated with 0.5 $\mu$Ci of 5,6,8,9,11,12,

14,15-[3H] Arachidonic Acid (sp act 100 Ci/mmol) in 1 ml of culture medium in 2054 culture tubes. After 24 hr at 37° C in a humidified atmosphere (5% CO in air) the labeled cells were washed twice by centrifugation (500g, 5 mins.). After removal of the medium, washed cells were solubilised in 1 ml of 0.1 N NaOH, the tubes were rapidly rinsed with the same volume of NaOH, and the pooled material was immediately acidified using 1 N HCl. Cellular lipids were extracted with chloroform-methanol as described by Vligh and Dyer. After evaporation under a stream of nitrogen at 30° C, the residues were dissolved in chloroform-methanol (200 µl) and applied to silica gel thin-layer plates (Whatman). The chromatograms were developed in hexanediethyl ether-formic acid (80:20:1) with lipid standards run in parallel. After exposure to iodide vapors, scrapped areas were counted using Picofluor 30 (Packard) as scintillation fluid.

Cell activation: Cells labeled with [3H] Arachidonic Acid (AA) for 24 hr were washed twice with RPMI (without serum) and kept in this medium for an additional 4 hr at 37° C in the presence of either Ca ionophore A23187, lipopolysaccharide (LPS) from Echerichia coli, zymosan from Saccharomyces cerevisiae yeast, silica (particle size 0.5-10 µm), or phorbol myristate acetate (PMA). In some experiments, cells were activated with interferon-(supernatant) of L12R4 cells treated with PMA for 48 hr. In addition, various amounts of virus coat protein (VCP) or serum albumin were added in test tubes. The proteins were dissolved in phosphate-buffered saline, pH 7.0. Equivalent volumes of buffer were added in control tubes.

Assay for prostaglandin and leukotriene synthesis: After the 4-hr incubation period, the medium (1.2 ml) and cell washes (1 ml) were pooled. The samples were brought to pH 3.0 with 70% citric acid. A 200-µl portion was counted to determine the total released H-labelled products and the remaining 2 ml was extracted twice to remove prostaglandin and unmetabolized AA as described by Humes et al. The aqueous phase was then reextracted with chloroform-methanol as described by Rouzer et al and Humes et al to obtain the leukotriene fraction. The ether and chloroform-methanol extracts were evaporated to dryness under a stream of nitrogen at 30° C. The residues from both extractions were dissolved, respectively, in 0.2 ml ethyl acetate-methanol (3:1) or chloroform. An aliquot (0.1 ml) was applied to lanes (1 x 20 cm) of thin-layer chromatography plates. Aliquots from the chloroform extracts were similarly spotted. The plates were developed with ethyl acetate-methanol-acetic acid (95:7:2), dried, and redeveloped in the same direction with hexaneethyl ether-acetic acid (60:40:1). The silica gel plates were then dried and the gel was scraped. Each 1 cm portion was harvested in scintillation vials and counted for radioactivity as described above. The relative percentages of peaks corresponding to prostaglandins, AA to be released by Arachidonic Acid, and leukotrienes were calculated.

Incorporation of labeled amino acids into the leukotriene fraction was obtained by incubation of cells for 24 hr in RPMI medium containing no cysteine and no glutamic acid. This medium was prepared using RPMI-select amine with replacement of RPMI vitamins by MEM vitamins. Leukotriene C labeled in the cysteine moiety was obtained by incubation of the cells for 24 hr in 1 ml of cysteine-free medium containing 5 µCI of L-[u-C]cysteine hydrochloride (35 Ci/mmol). Labeling with glutamic acid was similarly performed using glutamine-free medium and L-[U-C]glutamic acid (280 mCi/mmol).

## RESULTS

Incorporation of H-Labeled AA into Cellular Lipids:

When cells were cultured in the presence of tritiated AA, there was a linear incorporation of radioactivity into the cells over a 90-min period. The presence of VCPs (50-200 µg/ml) resulted in a concentration-dependent reduction of the rate of AA incorporation into macrophage cells. This significant reduction observed during a 90 min period became statistically nonsignificant when cells were maintained in the presence of [3H]AA for 24 hr. Indeed, at 24 hr, the total AA incorporated into cellular lipids was 241,000 cpm for controls and VCP treated cells, respectively.

The analysis of cellular lipids by thin-layer chromatography showed that more than 80% of AA was incorporated into phospholipids. The remaining radioactivity was distributed among free fatty acids (9%), cholesterol esters (3%), and triacylglycerol. The only difference between controls and virus coat protein-treated cells resided in the triacylglycerol fraction. After VCP treatment, 4.7% of C20:4 was found in triacylglycerol versus 1.23% in controls (P<0.01).

Release of AA and its Metabolites by Nonactivated Macrophage cells:

Nonactivated macrophage cells preincubated for 24 hours with tritiated AA and maintained after washing for an additional 4 hr in RPMI 1640 medium (without serum) released in the medium 7170 cpm. In the presence of virus macrophage cells (200 µg), the total release was raised to 10,530 cpm. The analysis of the released material (Table 2) showed that in the absence of VCP, PG represented the major labeled product.

## Table 2

## Arachidonic Acid Metabolism in Nonactivated Macrophages

| Treatment | Total Release | PG | AA | LT |
|---|---|---|---|---|
| No | 7,170 | 6384 89.12%) | 277 (3.87%) | 508 (7.0%) |
| VCP HIV gp120/ gp41 | 10,590 | 2,542 (24.01%) | 6632 (62.63%) | 1414 (13.35%) |

Note. Macrophage cells were prelabeled with tritiated Arachidonic Acid, washed, and incubated for 4 hrs without or with 200 μg VCP. Results are the means of two experiments performed in triplicate and are expressed in cpm and in percentage of radioactivity in each fraction after thin-layer chromatography. Abbreviations used: PG, prostaglandins; AA, Arachidonic Acid; LT, leukotrienes.

The effect of VCP was further investigated by using various concentrations of VCP and virus coat proteins from different species. Table 2 shows that virus coat protein at 100, 200 and 400 μg/ml resulted in a dose-dependent drop of PC synthesis from 32,000 cpm in controls to 8,724 cpm in cells treated with 400 μg virus protein. The unmetabolized AA was raised at 100 and 200 μg virus coat protein and then decreased to 14,165 cpm at 400 μg virus coat protein. This decrease was counter-balanced by a higher release of LT.

## LEUKOTRIENE SYNTHESIS IN MACROPHAGES

## TABLE 3

## Dose Response of VCP on Arachidonic Acid Metabolism

| Treatment (μg) | Total release | PG | AA | LT |
|---|---|---|---|---|
| No | 51,660 | 32.006 (61.96%) | 19,253 (37.27%) | 400 (0.77%) |
| VCP HIV/gp120/gp41 100 μg | 56,010 | 30,158 (53.84%) | 25,867 (46.18%) | 1,484 (2.65%) |
| VCP HIV/gp120/gp41 200 μg | 44,790 | 17,070 (38.11%) | 23,921 (53.40%) | 3,794 (8.47%) |
| VCP HIV/gp120/gp41 400 μg | 32,100 | 8,724 (27.18%) | 14,165 (44.13%) | 9,240 (28.79%) |

The present patent demonstrates that VCP is able to regulate AA metabolism in cell-free systems and intact cells. The present results indicate that AA metabolism is also regulated by VCP in macrophages. In macrophage, VCP reduces the uptake of AA by lowering the rate of incorporation of fatty acid into cellular phospholipids. Moreover, when the cellular phospholipid pool was prelabeled with [ H]AA, VCP induces an increased mobilisation of AA and greater release of H products. The major part of the tritiated released products was recovered as unmetabolized AA. The percentage of released AA converted into PG was reduced by VCP, while the LT fraction was increased. This VCP-medicated action was obtained both on nonactivated and on activated cells, irrespective of the activator used.

Measles Virus Coat Proteins in laboratory assay demonstrated binding affinity for Arachidonic Acid. HIV Viral Coat Proteins have also been shown to interact with Arachidonic Acid as outlined below. The importance of this, to the present patent, will be understood by those familiar with the relationship between the biochemistry of both Phospholipase A2 enzyme and Arachidonic Acid. (See Experiment A).

EXPERIMENT A

EVALUATION OF THE ARACHIDONIC ACID BINDING TO TWO PEPTIDES HIV gp120 AND p24

METHODS

Solubilisation of the peptides:
: Peptide gp120, 1 mg in 180 $\mu$l of an aqueous solution of acetic acid and 20 $\mu$l acetonitrile
: Peptide p24, 1 mg in 200 $\mu$l of an aqueous solution of acetic acid
Incubation of 50 $\mu$g or 100 $\mu$g of each peptide or 10 $\mu$g of pure rat Alpha Feto Protein with 270.000 DPM $^3$H C20:4 (SA 198 Ci/mmol), overnight at 4°C, in 0.5 ml of 10 mM potassium phosphate buffer 10mM pH 7.4.
Binding of Arachidonic Acid to peptides or protein measured after separation of the free ligand from the bound one using Lipidex 1000 (100 $\mu$l Lipidex solution 10 minutes at 4°C. The free ligand is bound to Lipidex 1000, the bound ligand to peptides and proteins remains in the supernatant after centrifugation.

RESULTS

Solubility of $^3$H C20:4 tested before and after addition of peptides or protein:

```
:     without peptide or protein                    70%
:     in presence of 10 µg pure rat AFP            100%
:     in presence of gp120:          50µg           65%
                                    100µg           90%
:     in presence of p24            50µg           23%
                                    100µg           30%
```

Binding of $^3$H C20:4 to peptides or protein after Lipidex 1000 treatment.

```
:     p24 peptide      :    0.8 x 10⁻⁶ DPM/mg
:     gp120 peptide    :    1.2 x 10⁻⁶ DPM/mg
:     Rat AFT          :   14.1 x 10⁻⁶ DPM/mg
```

CONCLUSIONS

1) In the presence of p24 peptide, there exists an important insolubility of the Arachidonic Acid.  * The Arachidonic Acid is bound to the glass vial. It was also demonstrated that Arachidonic Acid with HIV p24 peptides, bind negatively charged films or surfaces.

This phenomenon can be explained by either:

*This novel phenomenon associated with p24 binding of Arachidonic Acid/Glass complex may be used to remove HIV p24 antigens from patients' plasma or as a method to establish a quantitative assay for HIV p24 antigen levels in a plasma.

a) binding of the $^3$H C20:4 to the glass vial.

b) binding of the $^3$H C20:4 as a complex with the peptide to the glass vial.

2) A clear binding of C20:4 is observed with gp120 peptide.

This novel discovery of the similar action of AFP and certain viral coat proteins is further enhanced when one considers studies relating to vertical transmission of HIV. Our study would suggest that previous interpretations of such studies may be flawed by the fact that the peptide sequences of anti-serum to AFP may have been interpreted as the peptide gp120. In that the principal domain of gp120 reacts in an identical manner to the binding sites of AFP to AA. With this new knowledge, previous studies show that certain antibodies directed against AFP (reported as gp120) may confer protection from vertical transmission. Since HIV infected mothers who do not transmit the virus to their siblings have high titres to the HIV gp120 glycopeptide.

SUMMARY OF IN-VITRO ANTI-VIRAL TEST FOR AFP (HUMAN) ANTI-SERA ON C81 -66 CELLS INFECTED WITH HIV-RF.

NOTE:

1. The results are expressed as the number of wells positive for HIV p24 (out of the two replicates at each multiplicity of infection). Thus, 2 means that both wells contained viral antigen, while 0 means no virus was present.

2. Pretreatment means that the antibodies to Human Alpha feto protein were present during HIV virus absorption as well as afterwards.

3. An error in diluting the antibodies was made, so that following the first three concentrations (6,2, and 0.6 percent), an extra 10 fold dilution was made, so that the next dilution was 0.02% rather than 0.2%.

NOTE - GPP26 is the anti-serum developed in and purified from goat plasma to human alpha feto protein.

| Assay No. | | Drug | Conc. | Virus Replication at a.m.i. (log) of: | | | | | |
|---|---|---|---|---|---|---|---|---|---|
| | | | | -1 | -2 | -3 | -4 | -5 | -6 |
| + | 1 | GPP26 | 6 (%) | 2 | 2 | 0 | 0 | 0 | 0 |
| + | 2 | GPP26 | 2 | 2 | 2 | 0 | 0 | 0 | 0 |
| + | 3 | GPP26 | 0.6 | 2 | 2 | 2 | 0 | 0 | 0 |
| + | 4 | GPP26 | 0.02 | 2 | 2 | 2 | 2 | 0 | 0 |
| + | 5 | GPP26 | 0.006 | 2 | 2 | 2 | 2 | 1 | 1 |
| + | 6 | GPP26 | 0.002 | 2 | 2 | 2 | 2 | 2 | 0 |
| + | 7 | NHS* | 6 | 2 | 2 | 2 | 2 | 2 | 2 |
| + | 8 | NHS | 2 | 2 | 2 | 2 | 2 | 2 | 2 |
| + | 9 | NHS | 0.2 | 2 | 2 | 2 | 2 | 0 | 0 |
| + | 10 | NONE | | 2 | 2 | 2 | 2 | 2 | 0 |
| + | 11 | NONE | | 2 | 2 | 2 | 2 | 1 | 0 |
| − | 1 | GPP26 | 6 (%) | 2 | 2 | 0 | 0 | 0 | 0 |
| − | 2 | GPP26 | 2 | 2 | 2 | 0 | 0 | 0 | 0 |
| − | 3 | GPP26 | 0.6 | 2 | 2 | 2 | 0 | 0 | 0 |
| − | 4 | GPP26 | 0.02 | 2 | 2 | 2 | 2 | 1 | 0 |
| − | 5 | GPP26 | 0.006 | 2 | 2 | 2 | 2 | 1 | 0 |
| − | 6 | GPP26 | 0.002 | 2 | 2 | 2 | 2 | 2 | 0 |
| − | 7 | NHS | 6 | 2 | 2 | 2 | 2 | 1 | 0 |
| − | 8 | NHS | 2 | 2 | 2 | 2 | 2 | 0 | 0 |
| − | 9 | NHS | 0.2 | 2 | 2 | 2 | 2 | 2 | 0 |
| − | 10 | NONE | | 2 | 2 | 2 | 2 | 2 | 0 |
| − | 11 | NONE | | 2 | 2 | 2 | 2 | 2 | 0 |
| − | 12 | AZT | 10 μM | 2 | 1 | 0 | 0 | 0 | 0 |
| − | 13 | AZT | 1 | 2 | 2 | 2 | 1 | 2 | 2 |
| − | 14 | AZT | 0.1 | 2 | 2 | 2 | 2 | 0 | 0 |
| − | 15 | AZT | 0.01 | 2 | 2 | 2 | 2 | 1 | 0 |

*NHS = normal horse serum
+ Indicates pretreatment
− Indicates no pretreatment

In the meantime, observation of the cultures for cytopathic effects (the RF strain of HIV rapidly produces large numbers of giant cells and syncytia in this cell line) indicates that the GPP26, at concentrations between 6% and 0.006%, significantly inhibited the development of such cells. Control serum had no significant effect in this assay.

Although the present invention has been described in connection with preferred embodiments, it will be appreciated by those skilled in the art that additions, modifications, substitutions and deletions not specifically described may be made without departing from the spirit and scope of the invention defined in the appended claims.

**Claims**

1. Use of an antigenic peptide whose immune response, alone or with the assistance of an adjuvant, generates antibodies which bind with specific viral coat proteins or glycoproteins, said viral coat proteins or glycoproteins existing either on free virus in the plasma or expressed on cell membrane of infected host cells, and said viral coat proteins expressing amino acid sequences which bind to Arachidonic Acid, for the manufacture of pharmaceutical formulations for treatment of a patient suffering from infection by and/or disease caused by virus, mycoplasma and/or bacteria.

2. Use of an antigenic peptide whose immune response, alone or with the assistance of an adjuvant, generates antibodies which bind with specific amino acid sequences on cell membrane of mycoplasma,

EP 0 468 441 A1

said amino acid sequences being capable of binding mycoplasma to Arachidonic Acid or to related fatty acid, either free or as components of host cell membrane, for the manufacture of pharmaceutical formulations for treatment of a patient suffering from infection by and/or disease caused by virus, mycoplasma and/or bacteria.

3. Use of an antigenic peptide whose immune response, alone or with the assistance of an adjuvant, generates antibodies which bind with specific amino acid sequences of bacterial cell membrane and/or bacterial cell walls, said amino acid sequences being capable of binding with Arachidonic Acid or related fatty acid, for the manufacture of pharmaceutical formulations for treatment of a patient suffering from infection by and/or disease caused by virus, mycoplasma and/or bacteria.

4. The use according to claim 1, 2 or 3, wherein said antigenic peptide is an Amino Acid sequence, in whole or in part, of human, animal or synthetic or recombinant Alpha Feto Protein, said amino acid sequence containing at least one binding site for Arachidonic Acid.

5. The use according to claim 1, 2 or 3, wherein said antigenic peptide is a peptide sequence which is similar to a peptide sequence which is active in the binding of human, animal or synthetic or recombinant alpha feto protein with (a) Arachidonic Acid, (b) polyunsaturated fatty acid having a greater affinity for binding AFP/HIV gp120/gp41/ gp160 type molecules than free or membrane bound Arachidonic Acid (AA) and/or (c) Omega-3 fatty acid.

6. The use according to claim 1, 2 or 3, wherein said antigenic peptide is a peptide sequence which is similar to a peptide sequence which is active in the binding of cell membrane of mycoplasma with (a) Arachidonic Acid, (b) polyunsaturated fatty acid having a greater affinity for binding AFP/HIV gp120/gp41/ gp160 type molecules than free or membrane bound Arachidonic Acid (AA) and/or (c) Omega-3 fatty acid.

7. The use according to claim 1, 2 or 3, wherein said antigenic peptide is a peptide sequence which is similar to a peptide sequence which is active in the binding of viral coat protein with (a) Arachidonic Acid, (b) polyunsaturated fatty acid having a greater affinity for binding AFP/HIV gp120/gp41/ gp160 type molecules than free or membrane bound Arachidonic Acid (AA) and/or (c) Omega-3 fatty acid.

8. The use as recited in any of claims 5, 6 or 7, wherein said Omega-3 fatty acid is selected from the group consisting of Eicosapentaenoic Acid and Docosahexaenoic Acid.

9. The use as recited in either of claims 7 or 8, wherein said viral coat protein is selected from the group consisting of HIV gp120, HIV p24, HIV gp41 and HIV gp160.

10. The use as recited in claim 9, wherein said gp120 sequence is Cys-Thr-His-Gly-Ile-Arg-Pro-Val-Val-Ser-Thr-Gln-Leu-Leu-Leu-Asn-Gly-Ser-Leu-Ala-Glu-.

11. The use recited in claim 9, wherein said p24 sequence is Ala-Asn-Pro-Asp-Cys-Lys-Thr-Ile-Leu-Lys-Ala-Leu-Gly-Pro-Ala-Ala-Thr-.

12. The use recited in claim 9, wherein said gp 41 sequence is selected from the group of sequences consisting of Leu-Gly-Leu-Trp-Gly-Cys-Ser-Gly-Lys-Ile-Cys, Leu-Gly-Ile-Trp-Gly-Cys-Ser-Gly-Lys-Leu-Ile-Cys, Leu-Gly-Ile-Trp-Gly-Cys-Ser-Gly-Lys-His-Ile-Cys, Leu-Gly-Met-Trp-Gly-Cys-Ser-Gly-Lys-His-Ile-Cys, and Leu-Asn-Ser-Trp-Gly-Cys-Ala-Phe-Arg-Gln-Val-Cys.

13. The use of a peptide sequence which binds an attachment site on Alpha feto protein for Arachidonic Acid for the manufacture of pharmaceutical formulations for treating a patient.

14. The use according to claim 13, wherein said peptide sequence is an Amino Acid sequence, in whole or in part, or combination of parts, of the Anti Serum to human, animal or synthetic or recombinant Alpha Feto Protein.

15. The use according to claim 13, wherein said peptide sequence is an Anti Serum to a peptide sequence which is active in the binding of human, animal or synthetic or recombinant alpha feto protein with (a)

17

Arachidonic Acid, (b) polyunsaturated fatty acid having a greater affinity for binding AFP/HIV gp120/gp41/gp160 type molecules than free or membrane bound Arachidonic Acid (AA), or (c) Omega-3 fatty acid.

16. The use according to claim 13, wherein said peptide sequence is an Anti Serum to a peptide sequence which is active in the binding of membrane of mycoplasma with (a) Arachidonic Acid, (b) polyunsaturated fatty acid having a greater affinity for binding AFP/HIV gp120/gp41/gp160 type molecules than free or membrane bound Arachidonic Acid (AA), or (c) Omega-3 fatty acid.

17. The use according to claim 13, wherein said peptide sequence is an Anti Serum to a peptide sequence which is active in the binding of viral coat protein with (a) Arachidonic Acid, (b) polyunsaturated fatty acid having a greater affinity for binding AFP/HIV gp120/gp41/gp160 type molecules than free or membrane bound Arachidonic Acid (AA), or (c) Omega-3 fatty acid.

18. The use as recited in any of claims 15, 16 or 17, wherein said Omega-3 fatty acid is selected from the group consisting of Eicosapentaenoic Acid and Docosahexaenoic Acid.

19. The use as recited in either of claims 17 or 18, wherein said viral coat protein is selected from the group consisting of HIV gp120, HIV p24, HIV gp41 and HIV gp160.

20. The use as recited in claim 19, wherein said gp120 sequence is Cys-Thr-His-Gly-Ile-Arg-Pro-Val-Val-Ser-Thr-Gln-Leu-Leu-Leu-Asn-Gly-Ser-Leu-Ala-Glu-.

21. The use recited in claim 19, wherein said p24 sequence is Ala-Asn-Pro-Asp-Cys-Lys-Thr-Ile-Leu-Lys-Ala-Leu-Gly-Pro-Ala-Ala-Thr-.

22. The use recited in claim 19, wherein said gp 41 sequence is selected from the group of sequences consisting of Leu-Gly-Leu-Trp-Gly-Cys-Ser-Gly-Lys-Ile-Cys, Leu-Gly-Ile-Trp-Gly-Cys-Ser-Gly-Lys-Leu-Ile-Cys, Leu-Gly-Ile-Trp-Gly-Cys-Ser-Gly-Lys-His-Ile-Cys, Leu-Gly-Met-Trp-Gly-Cys-Ser-Gly-Lys-His-Ile-Cys, and Leu-Asn-Ser-Trp-Gly-Cys-Ala-Phe-Arg-Gln-Val-Cys.

23. The use of at least one polyunsaturated fatty acid, said fatty acid having a greater affinity for binding AFP/HIV gp120/gp41/gp160 type molecules than free or membrane bound Arachidonic Acid (AA) for the manufacture of pharmaceutical formulations for treating a patient.

24. The use of Omega-3 fatty acids for the manufacture of pharmaceutical formulations for binding and removing or neutralizing infectious organisms or parts thereof which demonstrate the ability due to having specific amino acid sequence the capacity to bind Arachidonic Acid and/or other unsaturated fatty acids.

25. The use as recited in claim 24, wherein said Omega-3 fatty acid is selected from the group consisting of Eicosapentaenoic Acid and Docosahexaenoic Acid.

26. A method for preparing a pharmaceutical formulation for preventing transmission, infection or immunosuppression from virus, bacteria or mycoplasma and removal of free virus, bacteria or mycoplasma, comprising placing Arachidonic Acid or Omega-3 fatty acid moieties on an exposed surface of a liposome or other transport vehicle that can be carried in the blood plasma of a patient.

27. A method comprising providing a drug delivery system which targets infected cells with therapeutic agents, whereby mono/polyclonal antibodies to the Arachidonic acid binding sites of alpha feto protein/mycoplasma cell membrane are reconstituted into the bilayers of large liposomes containing the therapeutic agents therein.

28. The method recited in claim 27, wherein said liposomes bear anti serum to human, animal or synthetic or recombinant alpha feto protein.

29. A process comprising binding p24 with Arachidonic Acid/Glass complex to remove HIV p24 antigens from a plasma or to establish a quantitative assay for HIV p24 antigen levels in a plasma.

30. A method comprising administering as a treatment to blood plasma or transplant organs or stored semen, a formulation containing in whole or in part a peptide sequence which binds the attachment site on Alpha feto protein or HIV gp120/gp41/gp160/p24 for Arachidonic Acid or Omega-3 fatty acids for the removal of infectious organisms (viruses/bacterial/mycoplasma) either in the free state or as infected host cells.

31. A method according to claim 30, wherein the formulation is anchored to a support substrate or resin by which purification of an infected medium is effected.

32. The use according to claim 1, 2, or 3, wherein said antigenic peptide is composed of a recombinant fragment of human alpha feto protein with antigenic selectivity versus human albumin.

33. A method comprising altering DNA or RNA so as to cause synthesis of different coat glycopeptides unable to bind Arachidonic Acid or related unsaturated fatty acids.

34. A method for treating virus, mycoplasma or bacterial organism, comprising genetically manipulating DNA or RNA sequences of said virus, mycoplasma or bacterial organism so as to alter the glycopeptide sequences present in the coat or membrane of said virus, mycoplasma or bacterial organism, so as to inhibit the ability of said virus, mycoplasma or bacterial organism to bind Arachidonic Acid free or exposed on the host cell membrane, said alternating rendering virions and mycoplasma utilizing such host cell binding incapable of active attachment or infection.

35. Use of a human, animal or synthetic or recombinant amino acid sequence with or without adjuvant, for the manufacture of pharmaceutical formulations for producing an antibody response, said antibodies being mono or polyclonal which block and shield the attachment to binding sites exposed by infectious organisms or parts thereof for fatty acid, Arachidonic Acid, or associated Omega-3 fatty acid, and rendering further transmission and/or infection impossible.

36. The use of antigen and/or antibodies according to any one of claims 1-35 as prophylactic or therapeutic agents for the manufacture of pharmaceutical formulations for administration of antibodies directly or antigen induced, for treatment of diseases caused by viruses, mycoplasma, bacteria, or any combination thereof.

37. The use of compounds, according to any one of claims 1-36, for the manufacture of pharmaceutical formulations for administration prior to, while, or after administering anti-viral, antimycoplasmic or antibacterial agents to prevent immunosuppression which is otherwise caused when Arachidonic Acid binding sites present on viruses, mycoplasma or bacteria are released by the use of anti-viral, antimycoplasma or antibacterial agents.

38. The use of Arachidonic Acid or its analogues or other compounds, or combinations thereof, according to any one of claims 1-37, for the manufacture of pharmaceutical formulations for use as decoy molecules attached to the surface membranes of liposomes, red blood cells or other transport vehicles as therapeutic agents for the removal, by adherence, of active free virus, mycoplasma or bacteria, or combinations thereof, or infected host cells expressing viral coat proteins or glycopeptides.

39. The use of compounds according to claim 38 for the treatment outside the body of blood products, semen or organs.

40. The use of compounds which are inhibitors of phospholipase A2, cyclooxygenase and lipoxygenase in combination with compounds according to any one of claims 1-39 for the manufacture of pharmaceutical formulations for improving the immune status of a patient suffering immuno-malfunction as a result of viral and/or mycoplasma interference with the ratio and/or proportion of Arachidonic Acid metabolites produced.

41. A pharmaceutical formulation comprising an amino acid sequence, in whole or in part, of human, animal or synthetic or recombinant Alpha Feto Protein in the form of a gelatine capsule, tablet, dragee, syrup, suspension, topical cream, suppository, injectable solution, or in a kit for the preparation of a syrup, suspension, topical cream, suppository or injectable solution just prior to use, in silicon discs, polymer beads, or implants, or contained in a liposome.

42. A pharmaceutical formulation comprising a peptide sequence which is similar to a peptide sequence which is active in the binding of human, animal or synthetic or recombinant alpha feto protein with (a) Arachidonic Acid, (b) polyunsaturated fatty acid having a greater affinity for binding AFP/HIV gp120/gp41/gp160 type molecules than free or membrane bound Arachidonic Acid, and/or (c) Omega-3 fatty acid, in the form of a gelatine capsule, tablet, dragee, syrup, suspension, topical cream, suppository, injectable solution, or in a kit for the preparation of a syrup, suspension, topical cream, suppository or injectable solution just prior to use, in silicon discs, polymer beads, or implants, or contained in a liposome.

43. A pharmaceutical formulation comprising a peptide sequence which is similar to a peptide sequence which is active in the binding of cell membrane of Mycoplasma with (a) Arachidonic Acid, (b) polyunsaturated fatty acid having a greater affinity for binding AFP/HIV gp120/gp41/gp160 type molecules than free or membrane bound Arachidonic Acid, and/or (c) Omega-3 fatty acid, in the form of a gelatine capsule, tablet, dragee, syrup, suspension, topical cream, suppository, injectable solution, or in a kit for the preparation of a syrup, suspension, topical cream, suppository or injectable solution just prior to use, in silicon discs, polymer beads, or implants, or contained in a liposome.

44. A pharmaceutical formulation comprising a peptide sequence which is similar to a peptide sequence which is active in the binding of a viral coat protein with (a) Arachidonic Acid, (b) polyunsaturated fatty acid having a greater affinity for binding AFP/HIV gp120/gp41/gp160 type molecules than free or membrane bound Arachidonic Acid, and/or (c) Omega-3 fatty acid, in the form of a gelatine capsule, tablet, dragee, syrup, suspension, topical cream, suppository, injectable solution, or in a kit for the preparation of a syrup, suspension, topical cream, suppository or injectable solution just prior to use, in silicon discs, polymer beads, or implants, or contained in a liposome.

45. A pharmaceutical formulation comprising an amino acid sequence, in whole or in part, or combination of parts, of an Anti Serum to human, animal or synthetic or recombinant Alpha Feto Protein, in the form of a gelatine capsule, tablet, dragee, syrup, suspension, topical cream, suppository, injectable solution, or in a kit for the preparation of a syrup, suspension, topical cream, suppository or injectable solution just prior to use, in silicon discs, polymer beads, or implants, or contained in a liposome.

46. A pharmaceutical formulation comprising an Anti Serum to a peptide sequence which is active in the binding of human, animal or synthetic or recombinant alpha feto protein with (a) Arachidonic Acid, (b) polyunsaturated fatty acid having a greater affinity for binding AFP/HIV gp120/gp41/gp160 type molecules than free or membrane bound Arachidonic Acid, and/or (c) Omega-3 fatty acid, in the form of a gelatine capsule, tablet, dragee, syrup, suspension, topical cream, suppository, injectable solution, or in a kit for the preparation of a syrup, suspension, topical cream, suppository or injectable solution just prior to use, in silicon discs, polymer beads, or implants, or contained in a liposome.

47. A pharmaceutical formulation comprising an Anti Serum to a peptide sequence which is active in the binding of a cell membrane of Mycoplasma with (a) Arachidonic Acid, (b) polyunsaturated fatty acid having a greater affinity for binding AFP/HIV gp120/gp41/gp160 type molecules than free or membrane bound Arachidonic Acid, and/or (c) Omega-3 fatty acid, in the form of a gelatine capsule, tablet, dragee, syrup, suspension, topical cream, suppository, injectable solution, or in a kit for the preparation of a syrup, suspension, topical cream, suppository or injectable solution just prior to use, in silicon discs, polymer beads, or implants, or contained in a liposome.

48. A pharmaceutical formulation comprising an Anti Serum to a peptide sequence which is active in the binding of a viral coat protein with (a) Arachidonic Acid, (b) polyunsaturated fatty acid having a greater affinity for binding AFP/HIV gp120/gp41/gp160 type molecules than free or membrane bound Arachidonic Acid, and/or (c) Omega-3 fatty acid, in the form of a gelatine capsule, tablet, dragee, syrup, suspension, topical cream, suppository, injectable solution, or in a kit for the preparation of a syrup, suspension, topical cream, suppository or injectable solution just prior to use, in silicon discs, polymer beads, or implants, or contained in a liposome.

49. A pharmaceutical formulation as recited in any of claims 42, 43, 44, 46, 47 or 48 wherein said Omega 3

20

fatty acid is selected from the group consisting of Eicosapentaenoic Acid and Docosahexaenoic Acid.

50. A pharmaceutical formulation as recited in any of claims 44, 48 or 49, wherein said viral coat protein is selected from the group consisting of HIV gp120, HIV p24, HIV gp41 and HIV gp160.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 222 415 (RESEARCH CORP.)<br>--- | | A 61 K 39/00<br>A 61 K 39/12<br>A 61 K 39/21<br>A 61 K 37/02<br>A 61 K 39/395 |
| A | EP-A-0 343 258 (MITSUITOATSU CHEMICALS INC.)<br>--- | | |
| A | BIOLOGICAL ABSTRACTS, vol. 70, no. 8, 1980, abstract no. 51063, Philadelphia, PA, US; V. UTERMOHLEN et al.: "The effect of fatty acid addition in vitro on direct migration inhibition with paramyxoviral antigens", & CLIN. IMMUNOL. IMMUNOPATHOL. 16(3): 324-335. 1980<br>----- | | |

**TECHNICAL FIELDS SEARCHED (Int. Cl.5)**

A 61 K

The present search report has been drawn up for all claims

| Place of search | Date of completion of the search | Examiner |
|---|---|---|
| THE HAGUE | 15-10-1991 | REMPP G.L.E. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                     

& : member of the same patent family, corresponding document

EPO FORM 1503 03.82 (P0401)